Europäisches Patentamt

European Patent Office

Office européen des brevets

(19)

(11) Publication number: **0 165 606**
A2

(12) **EUROPEAN PATENT APPLICATION**

(21) Application number: 85107601.8

(22) Date of filing: 19.06.85

(51) Int. Cl.⁴: **C 12 Q 1/04**
C 12 P 1/00, C 12 R 1/89

(30) Priority: 22.06.84 JP 128510/84

(43) Date of publication of application:
27.12.85 Bulletin 85/52

(84) Designated Contracting States:
AT BE CH DE FR GB LI NL SE

(71) Applicant: CHLORELLA INDUSTRY CO., LTD
5th Floor, Toyokuni Building No. 4-6 Shiba Daimon
2-chome
Minato-ku Tokyo(JP)

(72) Inventor: Tanaka, Kuniaki
1575-1, Arakimachiaraki
Kurume-shi Fukuoka-ken(JP)

(72) Inventor: Konishi, Fumiko
1770-14, Arakimachishirakuchi
Kurume-shi Fukuoka-ken(JP)

(74) Representative: Strasse, Joachim, Dipl.-Ing. et al,
Strasse und Stoffregen European Patent Attorneys
Zweibrückenstrasse 17
D-8000 München 2(DE)

(54) **Testing agent for b cell blastogenesis.**

(57) A testing agent for B cell blastogenesis incudes a
substance having a molecular weight of 5,000 or more. The
substance is contained in an extract extracted from chlorella
alga using an aqueous solvent.

EP 0 165 606 A2

Croydon Printing Company Ltd.

- 1 -

## Testing agent for B cell blastogenesis

The present invention relates to a testing agent for B cell blastogenesis.

B cells are also called B lymphocytes. B cells are a type of lymphocyte present in peripheral lymphoid tissue such as in the spleen, lymphonodi or blood, and are antibody-forming cell precursors. In birds, B cell precursors in bone marrow stem cells ingress into the bursa Fabricii as a primary lymphoid organ and differentiate into B cells. However, a corresponding organ is unknown in Mammalia. B cells are distinguished from thymus-derived T cells which are another type of lymphocytes present in the above-mentioned lymphoid tissue in that the B cells have a humoral immunity function. B cells are stimulated by an external antigen and differentiate into plasma cells. The plasma cells produce an antibody specific to the external antigen. The higher the B cell function, i.e., the higher the function of differentiation into plasma cells upon being stimulated by a specific type of external antigen, the more antibody specific to the external antigen is produced. As a result, diseases by the external antigen tend not to be caused and if caused can be treated before becoming serious. For this reason, testing of B cell function is important in assessing immunological competence of a person against a specific antigen or resistance to a specific disease.

Concanavalin A (to be referred to as Con A hereinafter) prepared from Jack beans and lipopolysaccharide (to be referred to as LPS hereinafter) prepared from the outer membrane of gram-negative bacteria are known as lymphocyte function testing agents. However, they require complex manufacturing processes and are therefore expensive.

It is an object of the present invention to provide a testing agent for B cell blastogenesis which has a specificity to B cells and which is inexpensive.

In order to achieve the above object of the present invention, there is provided a testing agent for B cell blastogenesis comprising a substance contained in an extract extracted from an algae belonging to genus Chlorella and having a molecular weight of 5,000 or more as an effective component.

A testing agent for B cell blastogenesis according to the present invention specifically causes blastogenesis of normal B cells. In other words, the testing agent of the present invention promotes division and proliferation of normal B cells while causing limited blastogenesis of B cells having a weak function. For this reason, when a whole sample or a B cell-enriched fraction to be tested is treated with a testing agent according to the present invention, the degree of blastogenesis in B cells in the tested sample can be determined. This means that the B cell function can be tested.

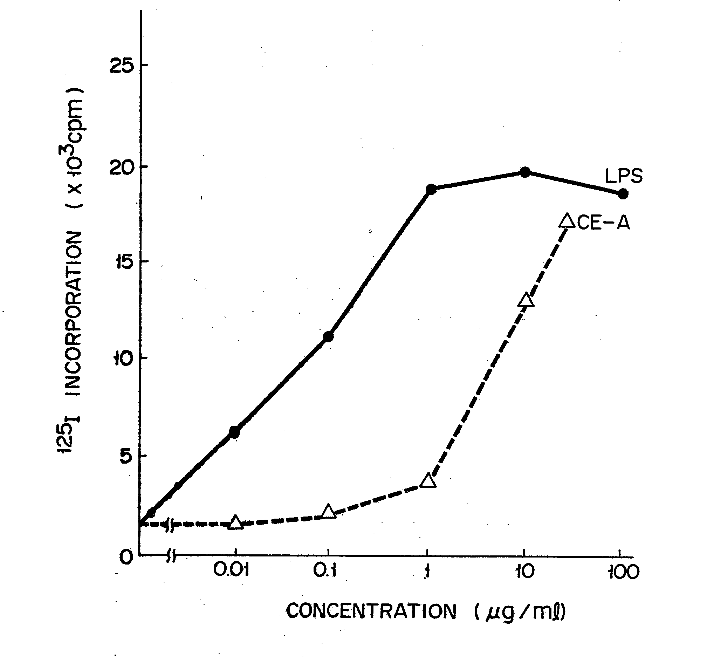

This invention can be more fully understood from the following detailed description when taken in conjunction with the accompanying drawing, in which:

The accompanying drawing is a graph showing the degree of blastogenesis in B cells with CE-A which is a testing agent for B cell blastogenesis according to the present invention and with LPS which is a known B cell blastogenesis agent.

A testing agent for B cell blastogenesis according to the present invention is an agent having as an effective agent a substance of a molecular weight of 5,000 or more which is contained in a chlorella extract.

This substance can be prepared in the following manner. First, chlorella as a raw material is prepared. Any chlorella of the genus Chlorella can be used (e.g., C. vulgaris, C. regularis, C. eripsoidia, etc.). The culture method of an alga of the genus Chlorella is well known. More specifically, a chlorella alga can be cultured by a method requiring sunlight, carbon dioxide gas and various nutrients, i.e., heteroculture. For example, a chlorella alga can be cultured by agitation in an outdoor pool containing acetic acid or glucose as a carbon source, urea or the like as a nitrogen source, and potassium phosphate as a K and P source.

The cultured chlorella alga is subjected to extraction by an aqueous solvent. Before extraction, the alga is preferably spray dried. That is, the alga is sprayed in a hot air flow at 120°C or higher and dehydrated. Then, the alga is subjected to extraction by an aqueous solvent. The aqueous solvent used contains water, an aqueous solution containing an acid (e.g., 0.2 N hydrochloric acid) or a base (e.g., 0.2 N sodium hydroxide) and having a pH of 5 to 9, and water-containing lower alcohols (e.g., methanol, ethanol. Although the spray-dried alga can be subjected to extraction by the aqueous solvent at 4 to 98°C for 15 minutes to 1 hour, it is preferably subjected to extraction by the aqueous solvent at 93 to 98°C for 15 to 40 minutes. The ratio of the alga and aqueous solvent during extraction can be selected to be, for example, 1 g of alga and 20 mℓ of aqueous solvent. In order to obtain an objective chlorella extract (to be referred to as CE hereinafter), the solvent extract is centrifuged to recover the CE as supernatant.

The thus obtained CE can be directly used as

a testing agent for B cell blastogenesis. However, in order to improve the effect as a testing agent, a fraction having a molecular weight of 5,000 or more (to be referred to as CE-A hereinafter) is preferably recovered from the CE by purification. CE-A having a molecular weight of 5,000 or more can be obtained by gel filtration chromatography or by dialysis using water. CE-A contains various kinds of polysaccharides and proteins. When gel filtration chromatography is performed, well known Sephadex G-25 (trade name) can be used. CE-A is obtained through this purification process.

The CE or CE-A obtained in this manner is used for testing the function of B cells. Before testing, the CE or CE-A need not be subjected to any treatment. However, the CE or CE-A as obtained may be lyophilized for purpose of easy handling. The function of B cells can be tested by allowing the CE or CE-A to act on the B cells and examining the degree of blastogenesis. The degree of blastogenesis in B cells can be determined by incubating B cells and a marker in the presence of CE or CE-A and measuring the amount of the marker incorporated into the B cells after incubation. The marker can be a radiation-marked nucleoside such as $^{125}$IUdR which is used for synthesis of nucleic acids.

The testing procedure of the function of B cells using a testing agent for B cell blastogenesis according to the present invention will be described in detail below. Three milliliters of a Ficoll-Hypaque separation medium for lymphocytes are charged in a small test tube, and 2 ml of sample blood diluted in 2 ml of phosphate buffered saline (PBS) (a total of 4 ml) are poured thereover so as not to cause agitation. When the resultant solution is centrifuged at 400 g for 30 minutes, red blood cells collect at the bottom of the test tube while the lymphocytes collect at the boundary between the Ficoll-Hypaque separation medium and its serum. The lymphocytes are recovered. The recovered lymphocytes contain T and B cells. The lymphocytes are suspended to have a cell concentration of $2 \times 10^{16}$ - $10 \times 10^{16}$ cells/m$\ell$, usually $5 \times 10^{6}$ cells/m$\ell$, using RPMI1640 as a culture medium. 100 μ$\ell$ of the lymphocytes suspension are mixed with 100 μ$\ell$ of a solution of the agent in RPMI 1640, containing 10 - 100 μg of lyophylized CE or CE-A per m$\ell$ of RPMI1640. The resultant mixture is incubated in a carbon dioxide incubator at 37°C for 42 hours. After 20 μ$\ell$ of 20 μCi/m$\ell$ of RPMI1640 medium of $^{125}$IUdR are added to the mixture and incubation is performed for another 6 hours, the amount of $^{125}$IUdR incorporated in the B cells is measured.

Example 1

Preparation of Testing Agent for B cell blastogenesis

Chlorella vulgaris was cultured by conventional heteroculture. The culture was performed under agitation in an outdoor pool using acetic acid or glucose as a carbon source, urea or the like as a nitrogen source, and potassium phosphate as a K and P source. The collected chlorella alga was spray-dried in a hot air flow at 120°C. One gram of the dried chlorella alga was placed in 20 m$\ell$ of water at 93°C and was heated within a temperature range of 93 to 98°C for 15 minutes under frequent agitation. Thereafter, the water containing the alga was cooled to 4°C, and centrifuged at 6,000 rpm for 15 minutes. The supernatant was recovered to obtain CE. The CE was gel-filtrated with Sephadex G-25 (trade name) to obtain a fraction (CE-A) having a molecular weight of 5,000 or more.

Example 2

Lymphocyte Blastogenesis Capacity of CE-A

Spleen cells of a C3H/HeN mouse were suspended in RPMI1640 culture medium* added with 5% fetal calf serum and 2-mercaptoethanol such that the lymphocytes had a cell concentration of $5 \times 10^6$ per milliliter.
100 µℓ of the suspension were mixed with 100 µℓ of each
solution of CE-A obtained in Example 1 diluted to
various concentrations using the above-mentioned medium.
As a control, mixtures of 100 µℓ of the lymphocyte
suspension prepared above with 100 µℓ of each solution
of LPS as a known B cell blastogenesis agent (derived
from E. coli and available from Biological Laboratories,
Inc., California, U.S.A.) diluted to various concentrations using the above-mentioned medium were prepared.
Each mixture was placed on a microplate and was
incubated at 37°C in a carbon dioxide gas incubator.
The suspension containing only the lymphocytes was also
cultured under the same conditions. Six hours before
measurement, 20 µℓ of 20 µCi/mℓ of the medium of $^{125}$IUdR
were added to each microplate. The amount of $^{125}$IUdR in
lymphocytes was measured 24 hours, 48 hours and 72 hours
after the culture was started. To measure the amount of
$^{125}$IUdR incorporated in cells, the cells were adhered to
filter papers. After drying the papers, the amount of
$^{125}$IUdR contained in the papers was measured using a
gamma scintillation counter. The obtained results are
shown in Table 1 and in the accompanying drawing. Each
value represents an average value of 6 samples.

Table 1

Amount of $^{125}$IUdR incorporated in cells ($\times 10^3$ cpm)

| Blastogenesis agent (µg/mℓ) | 24 hours (SI**) | 48 hours (SI) |
|---|---|---|
| No agent added | 1.0+0.3 (1.0) | 2.7+0.2 (1.0) |
| LPS(10) | 5.6+0.8 (5.6) | 9.5+0.3 (3.6) |
| CE-A(30) | 4.1+0.4 (4.1) | 7.6+0.2 (2.8) |

As can be seen from Table 1 above, when the incorporation of [125]IUdR in C3H/HeN mouse spleen cells was examined in vitro, in a sample containing CE-A, the incorporation increased more than with a sample containing no agent although the amount was slightly smaller than in a sample containing LPS. This demonstrates that blastogenesis of lymphocytes occurred in the sample containing CE-A. It can also be seen from the table that the incorporation peak is 48 hours after the culture is started. Further, as can be seen from the drawing, the activity of the agent is concentration dependent.

| * Composition of RPMI1640 medium | (mg/mℓ) |
|---|---|
| L-arginine·HCℓ | 200 |
| L-asparagine | 50.0 |
| L-aspartic acid | 20.0 |
| L-cysteine | 50.0 |
| L-glutamine | 300 |
| L-glutamic acid | 20.0 |
| Glycine | 10.0 |
| L-histidine·HCℓ | 15.0 |
| Hydroxy-L-proline | 20.0 |
| L-leucine | 50.0 |
| L-isoleucine | 50.0 |
| L-lysine·HCℓ | 40.0 |
| L-methionine | 15.0 |
| L-phenylalanine | 15.0 |
| L-proline | 20.0 |
| L-serine | 30.0 |
| L-threonine | 20.0 |
| L-tryptophan | 5.0 |
| L-tyrosine | 20.0 |
| L-valine | 20.0 |
| L-paraminobenzoate | 1.0 |
| Biotine | 0.2 |
| Calcium pantothenate | 0.25 |
| Choline chloride | 3.0 |

| | |
|---|---|
| Folic acid | 1.0 |
| Inositol | 35.0 |
| Nicotinamide | 1.0 |
| Pyridoxal·HC$l$ | 1.0 |
| Riboflavin | 0.2 |
| Thiamine·HC$l$ | 1.0 |
| Vitamin B12 | 0.005 |
| Glucose | 2000 |
| Glutathione | 1.0 |
| NaC$l$ | 6000 |
| KC$l$ | 400 |
| $MgSO_4 \cdot 7H_2O$ | 100 |
| $Na_2HPO_4 \cdot 7H_2O$ | 1512 |
| $Ca(NO_3)_2 \cdot 4H_2O$ | 100 |
| Phenol red | 5.0 |

** SI: Stimulation Index, the value in parentheses shows ratio of an amount of [125]IUdR incorporated in each sample to 1.0 of the control sample containing no agent.

<u>Example 3</u>

<u>B cell Blastogenesis Function of CE-A</u>

Although it has been confirmed that CE-A causes blastogenesis in lymphocytes, which type of T or B lymphocytes caused blastogenesis has not been determined.  This Example was performed to determine this.

C3H/HeN spleen cells were treated with anti-thy 1.2[+] antibody and complement to damage T cells.  The same test as in Example 2 was performed using the lymphocytes consisting of only B cells obtained in this manner.  The obtained results are shown in Table 2 below.

## Table 2

Amount of $^{125}$IUdR incorporation after 48hr culture in spleen cells treated with anti-thy $1.2^+$ antibody and complement (x $10^3$ cpm)

| Blastogenesis agent (μg/mℓ) | Non-treated spleen cells (SI) | Spleen cells treated with anti-thy $1.2^+$ antibody (SI) |
|---|---|---|
| No agent added | 2.7±0.2 (1.0) | 0.7±0.1 (1.0) |
| LPS(10) | 9.5±1.2 (3.56) | 12.7±2.1 (17.5) |
| CE-A(30) | 5.7±0.5 (2.13) | 4.5±0.3 (6.3) |

As can be seen from Table 2, the activity of CE-A is not lost even though a sample with damaged T cells is used, and this reveals that CE-A is a blastogenesis agent having a specificity to B cells.

The same test as in Example 2 was performed using C3H/HeN mouse thymus cells without B cells. For the purpose of comparison, the amount of $^{125}$IUdR incorporated in a sample was also measured for Con A as a T cell blastogenesis agent. The obtained results after culture for 48 hours are shown in Table 3 below.

## Table 3

| Blastogenesis agent (μg/mℓ) | Amount of $^{125}$IUdR incorporated in (x $10^3$ cpm) | SI |
|---|---|---|
| No agent added | 0.1±0.0 | 1.0 |
| Con A(5) | 3.7±0.6 | 31.8 |
| LPS(10) | 0.3±0.1 | 2.2 |
| CE-A(10) | 0.1±0.0 | 1.2 |

As can be seen from Table 3, Con A caused a high degree of blastogenesis in thymus cells consisting of T cells, but LPS caused far less blastogenesis in thymus cells.  However, CE-A caused almost no blastogenesis in thymus cells.  Therefore, it is seen that CE-A is a blastogenesis agent having a specificity to B cells.

Claims:

1. A testing agent for B cell blastogenesis comprising as an effective component a substance having a molecular weight not less than 5,000 contained in an extract extracted from a chlorella alga by an aqueous solvent.

2. A testing agent according to claim 1, characterized in that the substance is extracted in the aqueous solvent at 4 to 98°C for 15 minutes to 30 hours.

3. A testing agent according to claim 2, characterized in that the aqueous solvent is water and extraction is performed at 93 to 98°C for 15 to 40 minutes.

4. A testing agent for B cell blastogenesis comprising as an effective component a substance having a molecular weight of not less than 5,000 which is obtained by extraction of a chlorella alga by an aqueous solvent and purifying the extract by gel filtration chromatography or by dialysis against water.